# EUROPEAN PATENT APPLICATION

(11) **EP 3 238 603 A1**
(43) Date of publication of application: **01.11.2017**
(21) Application number: 15872565.5
(22) Date of filing: 18.11.2015
(51) Int. Cl.: A61B 1/00, G02B 23/24

(54) **ENDOSCOPE**

(30) Priority: 25.12.2014 JP 2014262651
(71) Applicant: Olympus Corporation, Tokyo 192-8507 (JP)
(72) Inventor: TANII, Yoshiyuki, Tokyo 192-8507 (JP)
(74) Representative: Gunzelmann, Rainer
(86) International application number: PCT/JP2015/082414
(87) International publication number: WO 2016/103996

(57) **Abstract**

An endoscope (10) includes a substrate member (50) and a cable (60) including an electric wire (61), a shield member (67), and a drain cable (63). The drain cable (63) has electrical conductivity and a same outer diameter approximately as that of the electric wire (61). The drain cable (63) is electrically connected to the shield member (67) to electrically connect the shield member (67) to the substrate member (50), and is electrically separated from the electric wire (61).

## Description

### TECHNICAL FIELD

The present invention relates to an endoscope having a cable inside thereof.

### BACKGROUND ART

Generally, a cable provided inside of an endoscope comprises a plurality of electric wires, a shield member (an overall shield) that is provided outside of the electric wires and covers the electric wires, and a sheath that is provided outside of the shield member and covers the shield member. The shield member is formed by winding a plurality of metallic strands around each other.

When two cables are connected to each other, in one of the cables, an end portion of the shield member is pulled to the side of end portions of the electric wires and the end portions of the electric wires are exposed from the end portion of the shield member. The same applies to the other cable. The electric wires are connected by solder to form a first connected portion. The first connecting portion is covered by a heat-shrinkable tube for protection, and for insulation frommetallic components (not shown in the drawings) provided in the periphery of the first connecting portion. The shield members of the cables are also connected by solder on the side of the first connecting portion to form a second connecting portion. The second connecting portion is covered by a heat-shrinkable tube for protection and for insulation from metallic components (not shown in the drawings) provided in the periphery of the second connecting portion. Finally, the first connected portion in the electric wires and the second connected portion in the shield members are covered together by a heat-shrinkable tube for protection, for binding of the first and second connected portions, and for insulation from metallic components (not shown in the drawings) provided in the periphery of the first and second connected portions.

In the endoscope disclosed in Patent Literature 1, for example, the electric wires are electrically connected to each other by a connector portion implemented in a connecting member.

### CITATION LIST

### PATENT LITERATURE

Patent Literature 1: Jpn. Pat. Appln. KOKAI Publication No. 2006-288614

### SUMMARY OF INVENTION

### TECHNICAL PROBLEM

As a cable provided in the inside of a narrow member such as an endoscope, an operation of connecting shield members by solder is complicated, and such connecting is poorly assembled. If the number of connected portions between the shield members increases, complexity increases, and the assembly becomes worse. The solder causes ahardportion, the hard portion becomes longer, and causes a connected portion to be larger and thicker; as a result, a location where a connected portion is arranged would be limited. If components of a cable are thinned in diameter and thickness in order to downsize a connected portion, the durability of the connected portion will be degraded, the operationof connecting will become complicated, and the assembly may become worse. The same also applies to an electric connecting of a shield member and a substrate member.

The present invention has been achieved in view of the foregoing circumstances, and an object of the present invention is to provide an endoscope with improved assembly of electric connecting between a shield member and a substrate member.

### SOLUTION TO PROBLEM

One aspect of the endoscope of the present invention comprises: a substrate member that is capable of transmitting signals for operating the endoscope; a cable that is connected to the substrate member, the cable including: an electric wire that is electrically connected to the substrate member and is capable of transmitting the signals, the outer periphery of the electric wire having electrical insulation properties; a shield member that encapsulates an outer periphery of the electric wires to prevent leakage of the signals transmitted by the electric wires to an outside of the electric wires, and that has electrical conductivity; and a drain cable that has electrical conductivity and an outer diameter approximately the same as that of the electric wire, that is electrically connected to the shield member to electrically connect the shieldmember to the substrate member, and that is electrically separated from the electric wires.

### ADVANTAGEOUS EFFECTS OF INVENTION

According to the present invention, an endoscope with improved assembly in an electric connecting of a shield member and a substrate member can be provided.

### BRIEF DESCRIPTION OF THE DRAWINGS

FIG. 1 is a schematic view of an endoscope according to the first embodiment of the present invention.
FIG. 2A is a drawing explaining that a cable is connected to a substrate member provided inside of a control connector portion.
FIG. 2B is a horizontal sectional view taken along line 2B-2B shown in FIG. 2A.
FIG. 2C is a horizontal sectional view taken along line 2C-2C shown in FIG. 2A.
FIG. 2D is a drawing explaining that inner conductors and a drain cable are connected to a cable side connector portion via a terminal portion.
FIG. 3 is a drawing of the first modification of the first embodiment, particularly a cable wound around a relief portion according to the first modification.
FIG. 4A is a drawing of the second modification of the first embodiment, particularly a cable wound around a relief portion according to the second modification.
FIG. 4B is a side view showing the cable illustrated in FIG. 4A is wound around a pin portion.
FIG. 5A is a drawing of the third modification of the first embodiment, particularly a substrate member having a relief portion according to the third modification and being provided inside of a light connector portion, and electric wires connected to the substrate member.
FIG. 5B is a perspective view of the substrate member shown in FIG. 5A.
FIG. 6 is a drawing of the fourth modification of the first embodiment, to explain that a cable is connected to a substrate member provided inside of an operation portion.
FIG. 7A is a drawing illustrating a state where the cables are connected according to the second embodiment of the present invention, particularly the state where three cable side connector portions are provided, and one cable side connector portion is connected to the other two cable side connector portions.
FIG. 7B is a drawing illustrating a state where the cables are connected according to the second embodiment of the present invention, particularly the state where four cable side connector portions are provided and one cable side connector portion is connected to one of the other cable side connector portions.

### BRIEF DESCRIPTION OF EMBODIMENTS

Hereinafter, embodiments of the present invention will be described with reference to the drawings.

### [First Embodiment]

### [Configuration]

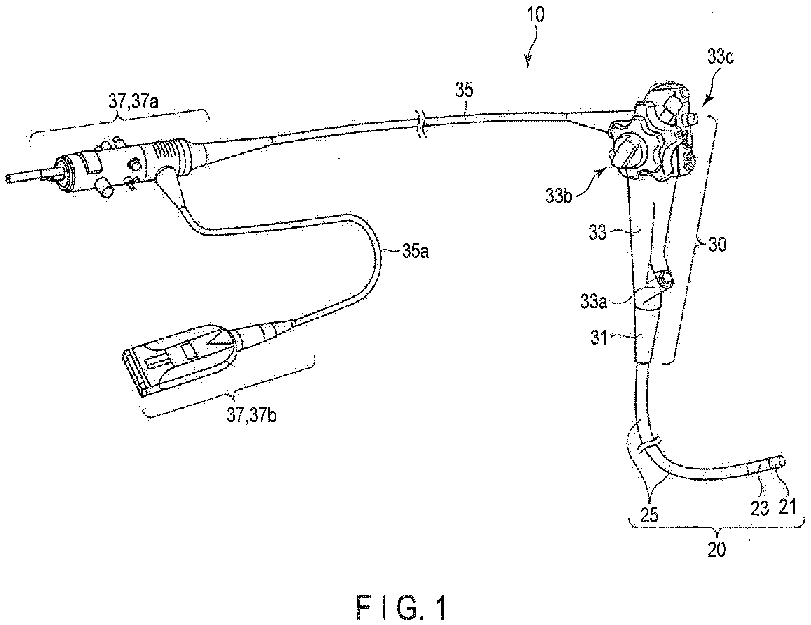

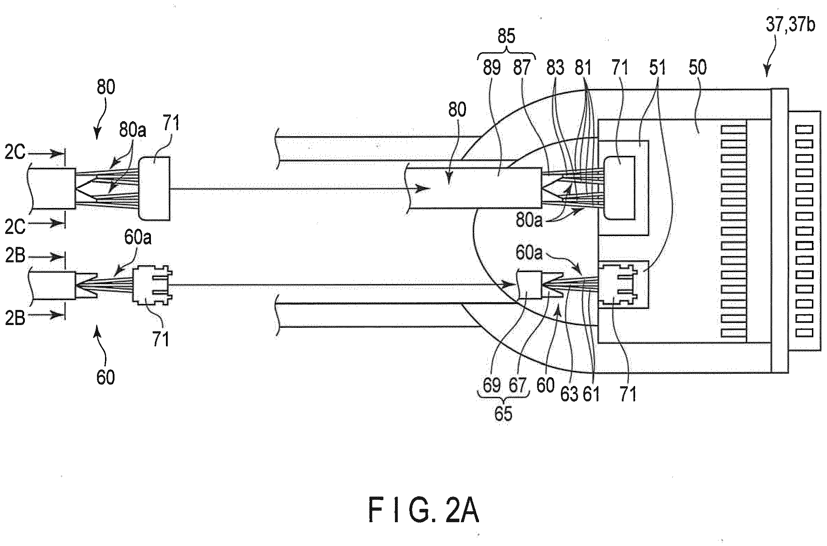

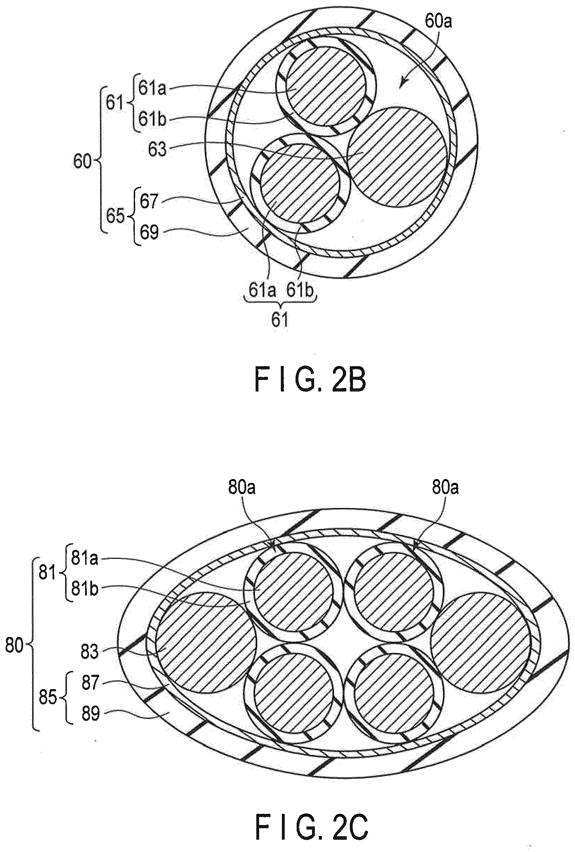

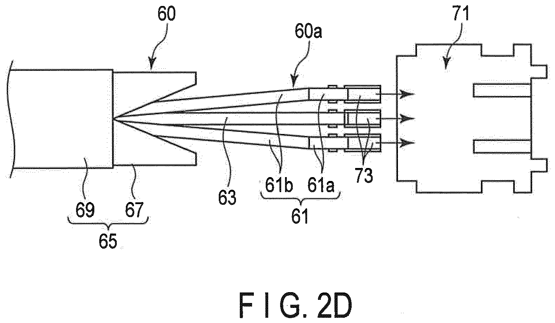

The first embodiment will be described with reference to FIGS. 1, 2A, 2B, 2C, and 2D. In some of the drawings, the illustration of some members is omitted for the sake of simplification.

### [Configuration 1 of Endoscope 10]

As shown in FIG. 1, the endoscope 10 comprises an elongated insertion portion 20 to be inserted into a body cavity, etc. of a patient, and an operation portion 30 coupled to a proximal end portion of the insertion portion 20 and configured to operate the endoscope 10.

### [Insertion Portion 20]

As shown in FIG. 1, the insertion portion 20 includes, from its distal end portion side to its proximal end portion side, a distal end hard portion 21, a bendable portion 23, and a flexible tube portion 25. A proximal end portion of the distal end hard portion 21 is coupled to a distal end portion of the bendable portion 23, and a proximal end portion of the bendable portion 23 is coupled to a distal end portion of the flexible tube portion 25.

The distal end hard portion 21 has an electronic component unit (not shown in the drawings) that is provided inside of the distal end hard portion 21. The electronic component unit includes, for example, a light emitting element, such as an LED which is provided in an illumination unit, and an imaging element, such as a CMOS that is provided in an imaging unit.

### [Operation Portion 30]

As shown in FIG. 1, the operation portion 30 includes a bending prevention portion 31 from which the flexible tube portion 25 extends, a grip portion (a main body) 33, which is gripped by an operator who operates the endoscope 10, coupled to a proximal end portion of the bending prevention portion 31, and a universal cord 35 connected to the grip portion 33 in a direction different from the direction in which the flexible tube portion 25 is connected.

### [Gripping Portion 33]

As shown in FIG. 1, the grip portion 33 includes a treatment instrument insertion portion 33a, a bending operation portion 33b which performs a bending operation on the bendable portion 23, and a switch portion 33c which is operated when various functions of the endoscope 10, such as air supply, water supply, suction, and image capturing, etc., are operated.

### [Universal Cord 35]

As shown in FIG. 1, the universal cord 35 includes a connector portion 37 connected to peripheral devices, which are not shown in the drawing, of the endoscope 10. The connector portion 37 includes a light connector portion 37a that is detachable from a light source device not shown in the drawings, which is a peripheral device, and a control connector portion 37b that is detachable from a control device not shown in the drawings, which is a peripheral device. The light connector portion 37a is provided at an end portion of the universal cord 35. A cord 35a is connected to and extends from a peripheral surface of the light connector portion 37a at the side of the light connector portion 37a. The control connector portion 37b is provided at the end portion of the cord 35b.

### [Configuration 2 of Endoscope 10, Substrate Member 30 and Cables 60 and 80]

As shown in FIG. 2A, the endoscope 10 includes a substrate member 50 provided inside of the endoscope 10, and cables 60 and 80 provided inside of the endoscope 10. Each of the cables 60 and 80 has a distal end portion indirectly connected to the substrate member 50 via a cable side connector portion 71, which will be described later, and a proximal end portion, which is not shown in the drawings, connected to the electronic component unit.

### [Substrate Member 50]

As shown in FIG. 2A, the substrate member 50 is provided inside of, for example, the connector portion 37. The substrate member 50 can transmit signals for operating the endoscope 10. In the present embodiment, the connector portion 37 is, for example, a control connector portion 37b. The substrate member 50 functions as an electric substrate that transmits predetermined signals and predetermined electric power between the substrate member 50 and the peripheral device, which is not shown in the drawings, and between the substrate member 50 and the cables 60 and 80, and that supplies the cables 60 and 80 with the predetermined signals and predetermined electric power supplied from the peripheral device. As described above, the substrate member 50 canbe electrically connected to a connecting member, which is not shown in the drawings, such as a control device as the peripheral device. The signals for operating the endoscope 10 are, for example, signals included in the above-described predetermined signals, and they are transmitted to the electronic component unit used to control the electronic component unit.

As shown in FIG. 2A, the substrate member 50 has a substrate side connector portion 51 connected to the cable side connector portion 71. The cable side connector portion 71 functions as a housing portion.

### [Cables 60 and 80]

The cables 60 and 80 as shown in FIG. 2A are arranged inside of the endoscope 10 from the distal end hard portion 21 to the connector portion 37. The cables 60 and 80 functions as anelectric cables that transmit predetermined signals and predetermined electric power between the cables 60 and 80 and the substrate member 50 and between the cables 60 and 80 and the electronic component unit, and that supply the electronic component unit with the predetermined signals and predetermined electric power supplied from the substrate member 50. If the cables 60 and 80 are illumination cables for an illumination unit, the electronic component unit is supplied with predetermined signals and predetermined electric power from the cables 60 and 80 to emit light. If the cables 60 and 80 are imaging cables for an imaging unit, the electronic component unit is supplied with predetermined signals and predetermined electric power from the cables 60 and 80 to capture images. In the present embodiment, signals are preferably transmitted between the connector portion 37 and the electronic component unit. For this reason, the cables 60 and 80 may be electrically connected to a different cable (not shown in the drawings) electrically connected to the electronic component unit via, for example, a connector portion (not shown in the drawings). Thus, signals can be transmitted between the connector portion 37 and the electronic component unit via the cables 60 and 80 and the different cable. The separate cable not shown in the drawings is arranged along with the longitudinal axis, as appropriate. The electronic component unit includes the light emitting element of the illumination unit and the imaging element of the imaging unit, which are arranged in the distal end hard portion 21.

Since the configuration of the cable 60 is approximately the same as the configuration of the cable 80, the configuration of the cable 60 will be used in the following explanation.

AsshowninFIGS. 2Aand2B, the cable 60 includes a plurality of electric wires 61, a drain cable 63, and a cover portion 65 that is provided outside of the electric wires 61 and the drain cable 63 to encapsulate outer peripheries of the electric wires 61 and an outer periphery of the drain cable 63. The drawing cable 63 is preferably wound with the electric wires 61.

As shown in FIG. 2B, the cover portion 63 includes a shield member (an overall shield) 67 provided outside of the electric wires 61 and the drain cable 63 to encapsulate the electric wires 61 and the drain cable 63, and a sheath (an electric insulator sheath) 69 provided outside of the shieldmember 67 to encapsulate an outer periphery of the shield member 67. The shield member 67 has electrical conductivity, and is made of metal, for example. The shield member 67 encapsulates the outer periphery of the electric wires 61 and the outer periphery of the drain cable. The sheath 69 has electrical insulation properties, and is made of resin, for example. One cable side connector portion 71 is provided at the end portion of the cable 60.

In the following, for the sake of explanation, the electric wires, the inner conductor, the cover member, the drain cable, the cover portion, the shield member, and the sheath of the cable 80 will be referred to as the electric wires 81, the inner conductor 81a, the cover member 81b, the drain cable 83, the cover portion 85, the shield member 87, and the sheath 89.

As shown in FIG. 2A and FIG. 2B, the cable 60 has one unit 60a, for example, and the unit 60a has two electric wires 61 and one drain cable 63.

As shown in FIG. 2A, the type of the cable 80 is different from the type of the cable 60, and the cable 80 includes two units 80a, for example, and each unit 80a has two electric wires 81 and one drain cable 83. Thus, the cable 80 has two pairs of electric wires 81 (i.e., four electric wires 81) and two drain cables 83. One cable side connector 71 is shared by the two units 80a. This cable side connector portion 71 is separate from the cable side connector portion 71 on the cable 60 side.

### [Electric Wires 61 and Drain Cable 63]

As shown in FIG. 2A and FIG. 2B, in the present embodiment, two electric wires 61 are provided in one cable 60, for example; however, the number of the electric wires is not limited thereto as long as more than one wire is provided. The electric wires 61 are flexible. The electric wires 61 are provided inside of the shield member 67.

The electric wires 61 are electrically connected to the substrate member 50, are capable to transmit signals to, for example, the substrate member 50. An outer surface of the electric wire 61 has electrical insulation properties. As shown in FIG. 2B, the electric wire 61 includes, for example, an inner conductor 61a electrically connected to the substrate member 50 so that the signals are transmitted between the inner conductor 61a and the substrate member 50, and a cover member (electric insulator sheath) 61b provided outside of the inner conductor 61a to encapsulate an outer periphery of the inner conductor 61a.

The inner conductor 61a is electrically connected to the substrate member 50 and the electronic component unit. Thus, the inner conductor 61a transmits predetermined signals between the cable 60 and the substrate member 50 and between the cable 60 and the electronic component unit, and supplies the electronic component unit with predetermined electric power supplied from the substrate member 50. Although not shown, the inner conductor 61a may include an electric wire bundle. The electric wire bundle is formed by winding a plurality of strands around each other. The cover member 61b has a cylindrical shape so that the inner conductor 61a is provided inside of the cover member 61b, and the outer peripheral surface of the inner conductor 61a is firmly adhered to an inner peripheral surface of the cover member 61b. The cover member 61b is provided along with the entire length of the inner conductor 61a.

The inner conductor 61a has electrical conductivity, and is made of metal, for example. The cover member 61b has electrical insulation properties, and is made of resin, for example. The cover member 61b electrically insulates the inner conductor 61a from metallic components provided outside of the inner conductor 61a, such as the drain cable 63 and the shield member 67.

As shown in FIG. 2B, the drain cable 63 has an outer diameter approximately the same as that of the electric wire 61. Since the cover member 61b is thinner than the inner conductor 61a, it can be assumed that the drain cable 63 has an outer diameter approximately the same as that of the inner conductor 61a. The drain cable 63 has electrical conductivity, and is made of metal, for example. The drain cable 63 is provided inside of the shield member 67. The drain cable 63 is inserted through the shield member 67. The drain cable 63 is electrically connected to the shield member 67, so that the electric potential of the drain cable 63 comes to be at the electric potential of the shield member 67. For this reason, an outer peripheral surface of the drain cable 63 is connected to an inner peripheral surface of the shield member 67. Although continuous electric connecting of the drain cable 63 from a distal end to a proximal end of the shield member 67 is preferable, it is not always necessary. For example, at least a part of the outer peripheral surface of the drain cable 63 may be electrically connected to at least a part of the inner surface of the shield member 67. Accordingly, the connecting between the drain cable 63 and the shield member 67 may be a point contact or a surface contact. The drain cable 63 is not necessarily provided along the entire length of the shield member 67. The drain cable 63 canbe electrically connected to the cable side connector portion 71, and the drain cable 63 should be electrically connected to the shield member 67. Thus, the drain cable 63 may be provided only at a distal end portion of the shield member 67 inside of the shield member 67 in such a manner that a distal end portion of the drain cable 63 is exposed from the distal end portion of the shield member 67. such a drain cable 63, the shield member 67 is electrically connected to the substrate member 50.

The drain cable 63 is electrically separated from the inner conductor 61a by the cover member 61b.

As shown in FIG. 2A and FIG. 2D, the distal end portions of the electric wires 61 and the distal end portion of the drain cable 63 are exposed (projected) from the distal end portion of the shield member 67 and a distal end portion of the sheath 69 along a longitudinal axis of the cable 60 toward the cable connector portion 71. This exposure shows that, for example, the distal end portions of the electric wires 61 and the distal end portion of the drain cable 63 are projected forward, from the distal end portion of the shield member 67 and the distal end portion of the sheath 69 toward the cable side connector portion 71 in the longitudinal axis of the cable 60, with respect to the distal end portion of the shield member 67 and the distal end portion of the sheath 69.

As shown in FIG. 2A and FIG. 2D, a distal end surface of the cable 60 is cut so that the distal end portions of the electric wires 61 and the distal end portion of the drain cable 63 are exposed from the distal end portion of the shield member 67 and the distal end portion of the sheath 69. Specifically, the distal end portion of the shield member 67 and the distal end portion of the sheath 69 are removed so that the distal end portions of the electric wires 61 and the distal end portion of the drain cable 63 are exposed. The length of the exposed distal end portion of the electric wire 61 is approximately the same as the length of the exposed distal end portion of the drain cable 63. The exposed distal end portions of the electric wires 61 and the exposed distal end portion of the drain cable 63 are separated from each other, and they are electrically separated as well.

As shown in FIG. 2D, in the exposed distal end portions of the electric wires 61, the distal end portions of the inner conductors 61a are exposed respectively from the distal end portions of the cover members 61b toward the cable side connector portion 71 in the direction of a longitudinal axis of the electric wires 61.

As shown in FIG. 2D, each of the exposed distal end portion of the inner conductor 61a and the exposed distal end portion of the drain cable 63 has an electric terminal portion 73 for electrical contact point. By inserting the terminal portions 73 into insertion opening portions, which is not shown in the drawings, of the cable side connector portion 71, the cable 60 is electrically connected to the cable side connector portion 71. The width of the cable side connector portion 71 is wider than the electric wires 61, the drain cable 63, and the cable 60. The cable side connector portion 71 has electrically and durability properties.

The exposed distal end portion of the drain cable 63 is covered by a cover member which is not shown in the drawings. The cover member has electrical insulation properties, and is made of resin, for example. The cover member has a heat-shrinkable tube, for example. The drain cable 63 is also electrically insulated by the cover member from metallic components, which is not shown in the drawings, provided in the periphery of the cable 60. The thickness of the cover member is approximately the same as the thickness of the cover member 61b.

The cover member may encapsulate the exposed distal end portions of the electric wires 61 and the exposed distal end portion of the drain cable 63 all together. The cover member may encapsulate the exposed distal end portions of the electric wires 61, the exposed distal end portion of the drain cable 63, the distal end portion of the shield member 67, the distal end portion of the sheath 69, and the cable side connector portion 71 all together.

### [Shield Member 67]

The shield member 67 as shown in FIGS. 2A, 2B, and 2D is formed by winding a plurality of metallic strands around each other. The shield member 67 has a reticular tubular shape, for example, a cylindrical shape. The shield member 67 covers the outer peripheries of the electric wires 61 and the outer periphery of the shield member 67 so that the electric wires 61 and the drain cable 63 are provided inside of the shield member 67.

The shield member 67 electrically protects the electric wires 61, so that noise caused by external devices, which is not shown in the drawings, such as a high-frequency treatment instrument for endoscope and an electrocautery, does not electrically affect the electric wires 61. In other words, the shield member 67 electrically shields the electric wires 61 from the external devices to prevent the electric wires 61 from being electrically affected by a high-frequency leak current generated in the external devices, etc. The external device is inserted from the treatment instrument insertion portion 33a, and is inserted through a channel (not shown in the drawings) that is continuous from the treatment instrument insertion portion 33a. The shield member 67 prevents leakage of the signals transmitted by the inner conductors 61a of the electric wires 61 to the outside of the electric wires 61. The shield member 67 is provided along the entire length of the inner conductor 61a. More specifically, the shield member 67 is provided from the distal end hard portion 21 through the connector portion 37, and is grounded to, for example, GND of the control device via the connector portion 37.

As shown in FIG. 2A and FIG. 2D, in the present embodiment, the distal end portion of the shield member 67 is exposed from the distal end portion of the sheath 69 toward the cable side connector portion 71 in the direction of a longitudinal axis of the shield member 67. This exposure shows that, for example, the distal endportionof the shieldmember 67 is projected forward, from the distal end portion of the sheath 69 toward the cable side connector portion 71 in the longitudinal axis of the shield member 67, with respect to the distal end portion of the sheath 69.

Herein, suppose the distal end surface of the sheath 69 in the cable 60 is cut so that the distal end portion of the shield member 67 is exposed from the distal end portion of the sheath 69. Specifically, the distal end portion of the sheath 69 is removed so that the distal end portion of the drain cable 67 are exposed. This cutting process is performed at the same time when the distal end portions of the electric wires 61 are cut to be exposed.

Although not shown, the distal end portion of the shield member 67 may be arranged at a same position as that of the distal end portion of the sheath 69, or it may be folded over in such a manner that it overlaps with the outer peripheral surface of the distal end portion of the sheath 69.

### [Sheath 69]

As shown in FIG. 2B, the sheath 69 has a tubular, for example, cylindrical shape, so that the electric wires 61, the drain cable 63, and the shield member 67 are provided inside the sheath 69. The sheath 69 is formed as a tube. The inner diameter of the sheath 69 is approximately the same as the outer diameter of the shield member 67, so that the inner periphery surface of the sheath 69 is firmly adhered to the outer periphery surface of the shield member 67.

### [Operation]

Although the cable 60 will be used in the following explanation, the operation and advantageous effects of the cable 80 are the same as those of the cable 60.

As shown in FIG. 2A, 2B, and 2D, the outer diameter of the drain cable 63 is approximately the same as that of the electric wire 61. Thus, by using, for example, the cable side connector portion 71 that is a three-line micro connector portion as shown in FIG. 2A, the distal end portion of the drain cable 63 can utilize the insertion opening portion of the cable side connector portion 71 into which the distal end portions of the inner conductors 61a are inserted, as shown in FIGS. 2A and 2D. Accordingly, the inner conductors 61a and the drain cable 63 can share the same cable side connector portion 71. At this time, the inner conductors 61a and the drain cable 63 can be appropriately arranged in the housing of the cable side connector portion 71. The type, shape, and size of the cable side connector portion 71 are selectable as needed in accordance with a purpose of use.

If the drain cable 63 is not provided in the cable 60, the distal end portion of the shield member 67 would be directly inserted into the insertion opening portion of the cable side connector portion 71. In this case, because of its cylindrical shape and thick outer diameter, the distal end portion of the shield member 67 cannot be inserted into the insertion opening portion of the cable side connector portion 71. It is necessary to narrow the distal end portion of the shield member 67 in order to be inserted into the insertion opening portion, but the narrowing process takes effort. However, in the present embodiment, the thin distal end portion of the drain cable 63 that is electrically connected to the shield member 67 and is thinner than the shield member 67 is utilized. Accordingly, the thin distal end portion of the drain cable 63 having the outer diameter approximately the same as that of the electric wire 61 is easily inserted into the insertion opening portion of the cable side connector portion 71. Thus, by using the drain cable 63, the shield member 67 is electrically connected to the substrate member 50 easily and securely via the drain cable 63 and the cable side connector portion 71, thereby improving the assembly of the electric connecting between the shield member 67 and the substrate member 50.

In the process of electrical connecting between the cable 60 and the substrate member 50, the terminal portion 73 is not directly fixed to the substrate member 50 by solder. In the process of electrical connecting, the substrate side connector portion 51 and the cable side connector portion 71 that is wider than the inner conductor 61a, the drain cable 63, and the terminal portion 73 are utilized to connect the cable side connector 71 to the substrate side connector portion 51 as shown in FIG. 2A. This connecting includes engagement of one of the cable side connector portion 71 and the substrate side connector portion 51 with the other, or coupling one to the other. As in the foregoing, the process of electrical connecting of the cable 60 to the substrate member 50 is easily performed only by connecting the cable side connector portion 71 to the substrate side connector portion 51. Thus, the assembly of the electric connecting between the shield member 67 and the substrate member 50 can be improved, and the connecting process can be performed by one action, and the durability of the connected portion can be secured without enlarging or elongating the connected portion. The connected portion includes the distal end portions of the electric wires 61 exposed from the shield member 67, the distal end portion of the drain cable 63 exposed from the shield member 67, the cable side connector portion 71, and the substrate side connector portion 51.

In the present embodiment, there is no need to use solder for the process of electrical connecting of the cable 60 to the substrate member 50. For this reason, issues with complicated procedures in the connecting process using solder, the poor assembly, the hard portion caused by soldering, and an elongated hard portion are resolved. Furthermore, a larger and thicker connected portion caused by solder is resolved, and a limitation on locations where a connected portion can be arranged is resolved.

In the not-shown cover member that encapsulates the exposed distal end portion of the shield member 67, the thickness of the cover member is approximately the same as the thickness of the cover member 61b. Accordingly, the connected portion that includes the distal end portions of the electric wires 61 exposed from the shield member 67 and the distal end portion of the drain cable 63 exposed from the shield member 67 is prevented from thickening even if the distal end portion of the drain cable 63 is covered by the cover member. In particular, the distal end portions of the electric wires 61 and the distal end portion of the drain cable 63 is ensured to be thinner than the cable side connector portion 71 and the substrate side connector portion 51.

As long as the cover member covers at least the distal end portion of the drain cable 63 , the process of attaching the cover member can be simply performed. Since one cover member is provided in one cable 60, the attaching process is performed for every cable 60, and the attaching process can be easily performed. The attaching process is performed before the cable side connector portion 71 is connected to the substrate side connector portion 51, and is not performed on the substrate member 50. Therefore, the attaching process can be carried out without any complexity.

When the cable side connector portion 71 is connected to the substrate side connector portion 51 as shown in FIG. 2A, although the shield member 67 is not directly electrically connected to the substrate member 50, the drain cable 63, which is electrically connected to the shield member 67, is electrically connected to the substrate member 50 via the cable side connector 71 and the substrate side connector 51. Thus, the shield member 67 is indirectly electrically connected to the substrate member 50 by the drain cable 63. Therefore, the assembly of the electric connecting between the shield member 67 and the substrate member 50 is improved.

### [Effects]

In the present embodiment, the shield member 67 can be electrically connected to the substrate member 50 via the drain cable 63 in an easy and secure manner, by using the drain cable 63 that is electrically connected to the shield member 67. Thus, the assembly of the electric connecting between the shield member 67 and the substrate member 50 can be improved in the present embodiment.

In the present embodiment, the outer diameter of the drain cable 63 is approximately the same as that of the electric wire 61. For this reason, the distal end portion of the drain cable 63 can be inserted into the insertion opening portion of the cable side connector portion 71 into which the distal end portions of the inner conductors 61a are inserted. Furthermore, the same cable side connector portion 71 which made in common to the inner conductors 61a and the drain cable 63 and the substrate side connector portion 51 can be utilized. Since the cable side connector portion 71 and the substrate side connector portion 51 canbe utilized, it is possible to easilyperform the electrical connecting of the cable 60 to the substrate member 50, to improve the assembly of the connecting, to perform the connecting process in one action, to prevent the connected portion from being enlarged and elongated, and to secure durability of the connected portion.

In the present embodiment, when electrically connecting the shield member 67 to the substrate member 50, the drain cable 63 that is electrically connected to the shield member 67 in the inside of the shield member 67 is used, and there is no need to use solder for the electric connecting process. Thus, it is possible to resolve the complexity in the connecting process that uses solder, to eliminate poor assembly, to prevent a hard portion caused by the solder, and to prevent the elongation of a hard portion. Furthermore, a larger and thicker connected portion caused by solder can be resolved, and a limitation on locations where a connected portion can be arranged can be prevented.

In the present embodiment, in the cover member (not shown in the drawings) which encapsulates the exposed shield member 67, the thickness of the cover member is approximately the same as the thickness of the cover member 61b. Accordingly, the connected portion that includes the distal end portions of the electric wires 61 exposed from the shield member 67, and the distal end portion of the drain cable 63 exposed from the shield member 67, can be prevented from thickening even if the distal end portion of the drain cable 63 is covered by the cover member. In particular, it is possible to make the distal end portions of the electric wires 61 and the distal end portion of the drain cable 63 is ensured to be thinner than the cable side connector portion 71 and the substrate side connector portion 51.

In the present embodiment, as long as the cover member encapsulates at least the distal end portion of the drain cable 63, the process of attaching the cover member can be simply performed. Since one cover member is provided in one cable 60, the attaching process can be performed for every cable 60, and the attaching process can be easily performed. If the attaching process is performed after the cable side connector portion 71 is connected to the substrate side connector portion 51, the complexity in the attaching process will increase. For this reason, the attaching process is performed before the cable side connector portion 71 is connected to the substrate side connector portion 51, and is not performed on the substrate member 50. Thus, the attaching process can be performed, solving complexity.

In the present embodiment, the substrate member 50 is provided inside of the connector portion 37, but is not limited thereto. If the proximal end portion of the insertion portion 20 is detachable from the bending prevention portion 31 of the operation portion 30, the cable side connector portion 71, for example, may be provided inside of the bending prevention portion 31, and the substrate member 50 that includes the substrate side connector portion 51 may be provided inside of the proximal end portion of the insertion portion 20. A location where the connected portion is to be arranged is not particularly limited.

### [First Modification of First Embodiment]

In the following, the first modification of the first embodiment, only the matters different from the first embodiment will be explained.

Generally, when the insertion portion 20 and the universal cord 35 are bent, the cable 60 provided inside of the insertion portion 20 and the universal cord 35 is moved. The movement means the cable 60 being twisted, bent, flexed from a straight state, straightened back from a flexed state, pulled, or pushed. Such movements may cause a load in a connecting portion of the cable 60 and the substrate member 50.

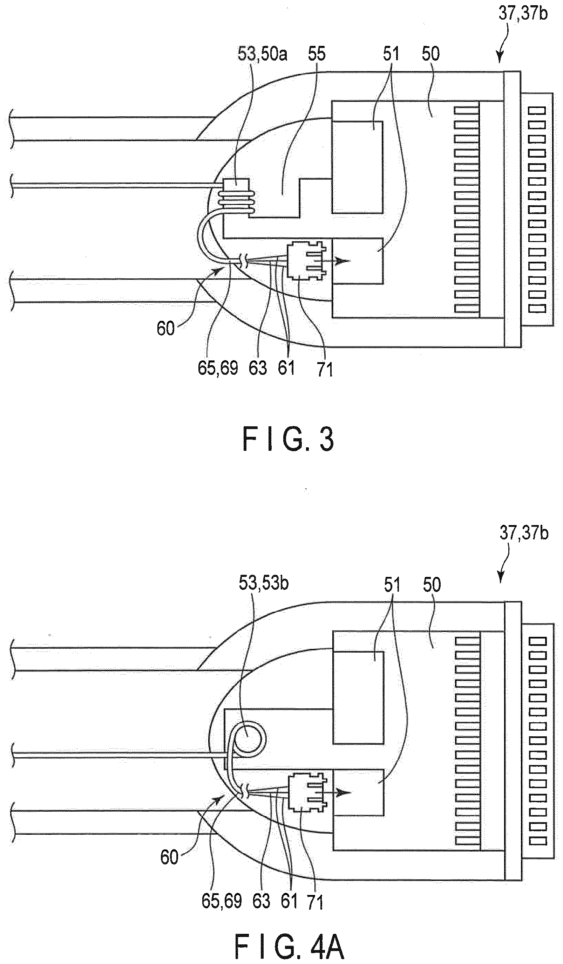

As shown in FIG. 3, the substrate member 50 includes a relief portion 53 that mitigates a load acting upon the connectedportion of the cable 60 and the substrate member 50 when the cable 60 moves. The substrate member 50 includes a cutout portion 55 which is formed by cutting out a part of the substrate member 50. The relief portion 53 includes a wound portion 50a around which the cable 60 is wound. The wound portion 50a is a part of the substrate member 50 around which a part of the cable 60 passing the cutout portion 55 is wound. The number of windings of the cable 60 around the wound portion 50a is not particularly limited. It is preferable to wind cable 60 loosely around the wound portion 50a, allowing some extent of play. The cable 60 may be closely wound around the wound portion 50a. A part of the cable 60 which is wound around the wound portion 50a is a middle portion existing in between the distal end portion and the proximal end portion of the cable 60, except for the connected portion. The cutout portion 55 penetrates the substrate member 50 in the direction of a thickness of the substrate member 50, and is formed by cutting out the substrate member 50 in the width direction of the substrate member 50.

Even when a load, such as a tensile stress, is applied to the cable 60, it is possible to mitigate the load directly acting upon the electric connected portion of the cable 60 and the substrate member 50, because the cable 60 is wound around the wound portion 50a.

If the cable 60 is loosely wound around the wound portion 50a having some play, the amount of play is increased or decreased in accordance with the movement of the cable 60. Thus, the relief member 53 can mitigate a load acting upon the cable 60.

### [Second Modification of First Embodiment]

In the following, the second modification of the first embodiment, and only the matters different from the first embodiment and the first modification will be explained.

The relief member 53 is not limited to what is explained in the first modification.

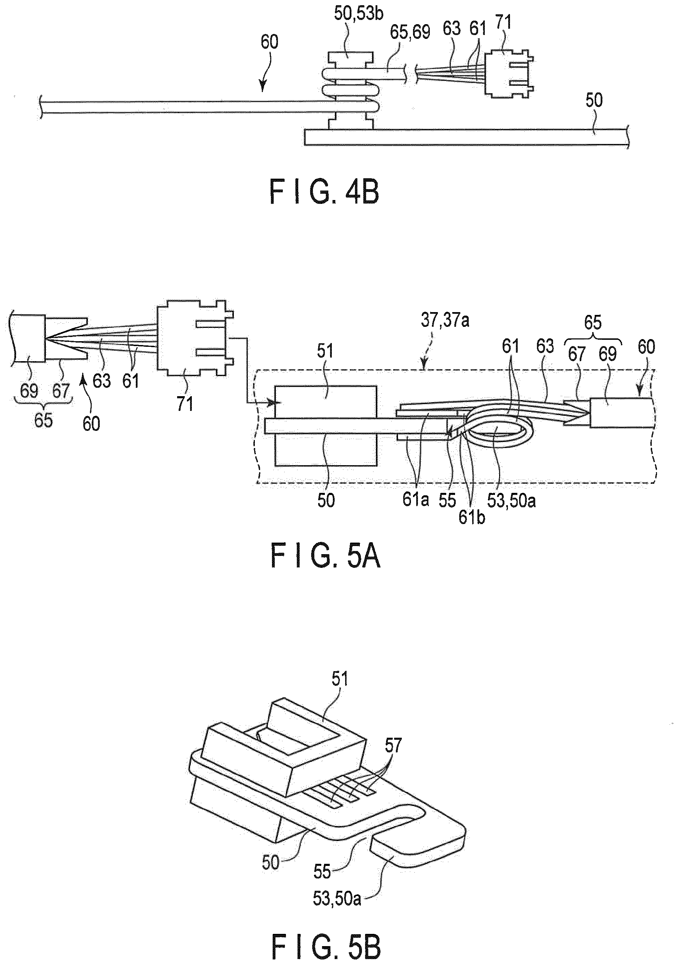

As shown in FIGS. 4A and 4B, the wound portion of the relief member 53 includes a pin portion 53b around which a part of the cable 60 is wound. The pin portion 53b is provided in the substrate member 50 to check a status of an electric circuit mounted on the substrate member 50, for example.

Accordingly, in the present modification, there is no necessity of processing the substrate member 50 itself, and a load acting upon the electric connected portion of the cable 60 and the substrate member 50 can be mitigated.

### [Third Modification of First Embodiment]

In the following, the third modification of the first embodiment, and only the matters different from the first embodiment and the first and second modifications will be explained.

In the first embodiment, an example where the substrate member 50 is provided inside of the control connector portion 37b is explained, but the first embodiment is not limited thereto. As shown in FIGS. 5A and 5B, the substrate member 50 maybe provided inside of the light connector portion 37a. The substrate member 50 can thus be provided at various locations in the inside of the connector portion 37.

If the substrate member 50 is provided inside of the light connector portion 37a, the substrate member 50 functions as an implemented substrate to which the distal end portions of the electric wires 61 being exposed from the distal end portion of the cover member 65 and the distal end portion of the drain cable 63 are electrically connected. Specifically, the inner conductors 61a, which are arranged in the right illustration in FIG. 5A and exposed from the cover member 61b, are directly connected to a plurality of electrodes 57 of the substrate member 50 (see FIG. 5B) by, for example, solder. Not the shield member 67 itself, which is arranged in the right illustration in FIG. 5A, but the drain cable 63 arranged in the right illustration in FIG. 5A is directly electrically connected to the electrodes 57. Thus, it is possible to more easily and securely achieve an electric connecting of the shield member 67 to the substrate member 50 via the drain cable 63 than in the case where the shield member 67 is directly electrically connected to the electrodes 57. At this time, the distal end portions of the electric wires 61 and the distal endportionof the drain cable 63 are electrically insulated from each other.

If the substrate member 50 is provided inside of the light connector portion 37a, the cable 60 which is connected to the substrate member 50 in advance (see the cable 60 arranged in the right illustration in FIG. 5A) is provided, for example, inside of the insertion portion 20 and the universal cord 35. Generally, when the insertion portion 20 and the universal cord 35 are bent, the cable 60 including the exposed electric wires 61 is moved. The movement means the cable 60 being twisted, bent, flexedfroma straight state, straightened back fromabent state, pulled, or pushed. Such movements may cause a load in a portion where the electric wires 61 (the inner conductors 61a) and the substrate member 50 are connected.

As shown in FIGS. 5A and 5B, the subject member 50 includes a relief portion 53 which mitigates a load acting upon the connected portion of the electric wires 61 and the substrate member 50 when the electric wires 61 are moved. The substrate member 50 includes a cutout portion 55 which is formed by cutting out a part of the substrate member 50. The relief portion 53 includes a wound portion 50a around which the electric wires 61 are wound. The wound portion 50a is a part of the substrate member 50, around which a part of the electric wires 61 exposed from the distal end portion of the cover portion 65 and passing the cutout portion 55 is wound. The number of windings of the electric wires 61 around the woundportion 50a is not particularly limited. It is preferable to wind the electric wires 61 loosely around the wound portion 50a, allowing some extent of play. The cable 60 may be closely wound around the wound portion 50a. A part of the electric wire 61 which is wound around the wound portion 50a is a middle portion existing in between the distal end portion and the proximal end portion of the electric wire 61, except for the connected portion. The cutout portion 55 penetrates the substrate member 50 in the direction of the thickness of the substrate member 50, and is formed by cutting out the substrate member 50 in the width direction of the substrate member 50. Although not shown, the inner conductors 61a exposed from the cover member 61b may be wound around the wound portion 50a.

In one cable 60 provided in the universal cord 35 (see the cable 60 provided in the right illustration in FIG. 5A), the electric wires 61 are wound around the wound portion 50a and directly fixed to the substrate member 50. The drain cable 63 is directly fixed to the substrate member 50. The drain cable 63 may be wound around the wound portion 50a and directly fixed to the substrate member 50.

By winding the electric wires 61 around the wound portion 50a, a load acting upon the electric connected portion of the electric wires 61 and the substrate member 50 can be mitigated.

If the electric wires 61 are loosely wound around the wound portion 50a having some play, the amount of play is increased or decreased in accordance with the movement of the electric wires 61. Thus, the relief member 53 can mitigate a load acting upon the electric wires 61.

If the substrate member 50 is provided inside of the light connector portion 37a, a cover member (not shown in the drawings) is provided. The cover member not shown in the drawings encapsulates the distal end portions of the electric wires 61 exposed from the cover member 65, the distal end portion of the drain cable 63 exposed from the cover member 65 , and the periphery portions of the cutout portion 55 and the wound portion 50a including themselves. The periphery portions may include a periphery of the distal end portions of the electric wires 61 and a periphery of the distal end portion of the drain cable 63. The cover member has electrical insulation properties, and is made of resin, for example. The cover member insulates the distal end portions of the electric wires 61, the distal end portion of the drain cable 63 and the periphery portions from metallic components provided in the peripheries thereof. The cover member has a heat-shrinkable tube, for example.

As shown in FIG. 5A and FIG. 5B, a substrate side connector portion 51 is provided in the substrate member 50. The substrate side connector portion 51 is electrically connected to the electrodes 57. The substrate side connector portion 51 is connected to the cable side connector portion 71, which is arranged in the left illustration in FIG. 5A. This cable side connector portion 71 is connected to the other cable 60 provided at the end portion of the light connector portion 37a (see the cable 60 arranged in the left illustration in FIG. 5A). By electrically fixing the drain cable 63 of the cable 60 that is arranged in the right illustration in FIG. 5A to the substrate member 50 via the electrodes 57, the shield member 67 of the cable 60 is electrically connected to the shield member 67 of the other cable 60 via the drain cable 63 that is arranged in the left illustration in FIG. 5A, to the cable side connector portion 71, to the substrate side connector portion 51, to the electrodes 57, and to the drain cable 63 that is arranged in the right illustration in FIG. 5A. Thus, the substrate member 50 according to the present modification functions as a relay substrate electrically connecting the cables 60.

### [Fourth Modification of First Embodiment]

In the following, the fourth modification of the first embodiment, only the matters different from the first embodiment and the first, second, and third modifications will be explained.

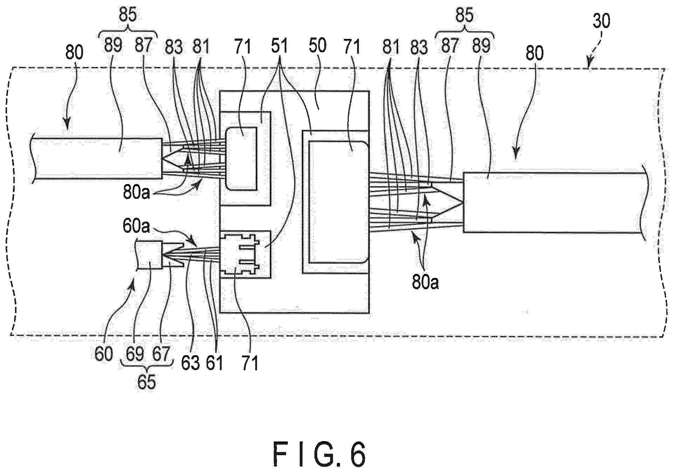

The substrate member 50 as shown in FIG. 6 is provided inside of the operation portion 30, for example, and may function as a relay substrate electrically connecting the cable 60 and the cable 80, or connecting the cables 80. In this case, the cable side connector portion 71 is detachable from the substrate member 50.

The substrate member 50 can thus be provided at various locations in the inside of the endoscope 10.

### [Second Embodiment]

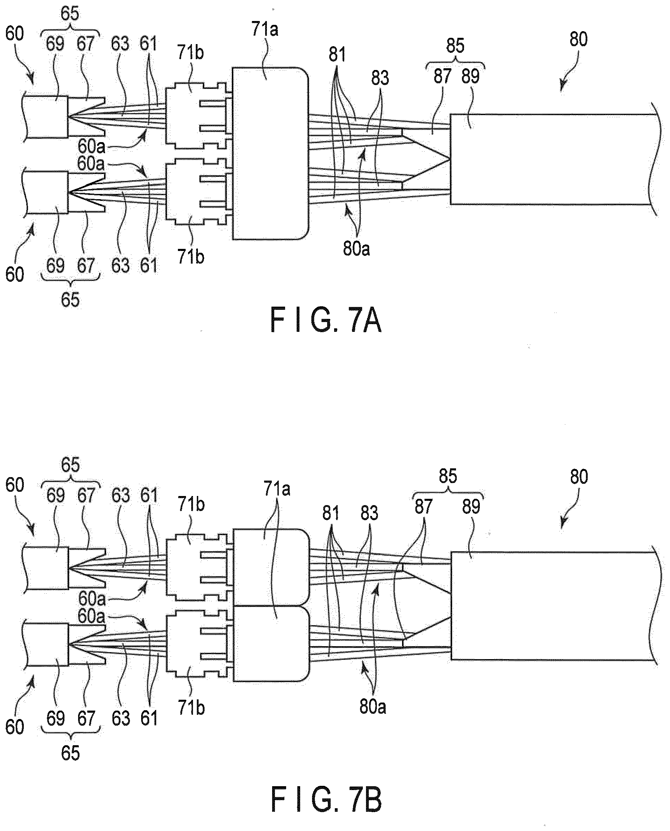

Only the matters different from the first embodiment will be described with reference to FIGS. 7A and 7B.

In the first embodiment, an example where the cable 60 is electrically connected to the substrate member 50 is explained, but the second embodiment is not limited thereto. The cable 60 and the cable 80 may be directly electrically connected to each other without the intervening substrate member 50. Since the electric connecting of the cable 60 and the cable 80 is not implemented in the substrate member 50, such electric connecting is performed in the air. A location where the connected portion is to be arranged is not particularly limited.

The cable 80 which is arranged in the right illustration in FIG. 7A and FIG. 7B has two units 80a, for example, and each of the units 80a has two electric wires 81 and one drain cable 83.

As shown in FIG. 7A, the female cable side connector portion 71a, which is connected to the cable 80, may be shared by the two units 80a. For this reason, one female cable side connector portion 71a is provided. As shown in FIG. 7A, the female cable side connector portion 71a is connected to two male cable side connector portions 71b, which are connected to the two cables 60 respectively.

As shown in FIG. 7B, for example, the female cable side connector portion 71a, which is connected to the cable 80, may be provided in each unit 80a. For this reason, two female cable side connector portions 71a are provided. As shown in FIG. 7B, each of the female cable side connector portions 71a is connected to each of the two male cable side connector portions 71a, which are respectively connected to the cables 60. In this case, one of the connected portions may be displaced from the other connected portion with respect to the direction of the longitudinal axis of the cable 60. Thus, the area peripheral to the connected portions can be prevented from being thickened.

Similar to the first embodiment, the exposed distal end portion of the drain cable 63 is covered by a cover member, which is not shown in the drawings. The cover member has electrical insulation properties, and is made of resin, for example. The cover member has a heat-shrinkable tube, for example. The drain cable 63 is insulated also by the cover member from metallic components, which is not shown in the drawings, provided in the periphery of the cable 60.

### [Modification of Second Embodiment]

The cable 60 may be connected to, for example, an imaging unit in the electric component unit, via the cable side connector portion 71. In this case, the electric wires 61 are electrically connected to an electric assembly included, for example, in the electric component unit.

The present invention is not limited to the above-described embodiments as they are, and can be embodied by modifying the structures without departing from the gist of the invention when being implemented. Various inventions can be formed by appropriate combinations of multiple structures disclosed in the above embodiments.

## Claims

1. An endoscope comprising:
a substrate member that is capable of transmitting signals for operating the endoscope; and
a cable that is connected to the substrate member, the cable including:
an electric wire that is electrically connected to the substrate member and is capable of transmitting the signals, an outer periphery of the electric wire having electrical insulation properties;
a shield member that encapsulates an outer periphery of the electric wire to prevent leakage of the signals transmitted by the electric wire to an outside of the electric wire, and that has electrical conductivity; and
a drain cable that has electrical conductivity and a same outer diameter approximately as that of the electric wire, that is electrically connected to the shield member to electrically connect the shield member to the substrate member, and that is electrically separated from the electric wire.

2. The endoscope according to claim 1, wherein the substrate member includes a relief portion that mitigates a load acting upon a connected portion of the cable and the substrate member when the cable moves.

3. The endoscope according to claim 2, wherein the relief portion includes a wound portion around which the cable is wound.

4. The endoscope according to claim 3, wherein the wound portion is a part of the substrate member, a part of the cable passing a cutout portion that is formed by cutting out the substrate member being wound around the wound portion.

5. The endoscope according to claim 3, wherein the wound portion has a pin portion around which a part of the cable is wound.

6. The endoscope according to claim 1, wherein the substrate member includes a relief portion that mitigates a load acting upon a connected portion of the electric wire and the substrate member when the electric wire move.

7. The endoscope according to claim 6, wherein the relief portion includes a wound portion around which the electric wire are wound.

8. The endoscope according to claim 7, wherein the wound portion is a part of the substrate member, a part of the electric wire passing a cutout portion that is formed by cutting out the substrate member being wound around the wound portion.

9. The endoscope according to claim 1, wherein the substrate member is provided inside of the connector portion of the endoscope.

10. The endoscope according to claim 1, wherein the substrate member is provided inside of an operation portion of the endoscope.

11. The endoscope according to claim 1, wherein
the electric wire includes:
an inner conductor that has electrical conductivity and is electrically connected to the substrate member so that the signals are transmitted between the inner conductor and the substrate member; and
a cover member that has electrical insulation properties and electrically separates the inner conductor and the drain cable from each other by encapsulating an outer periphery of the inner conductor.

12. The endoscope according to claim 1, wherein the drain cable is encapsulated by the shield member, and at least a part of an outer peripheral surface of the drain cable is electrically connected to at least a part of an inner peripheral surface of the shield member.

13. The endoscope according to claim 1, wherein the drain cable is in point contact or surface contact with the shield member.

14. The endoscope according to claim 1, wherein the drain cable is provided at a distal end portion of the shield member inside of the shield member in such a manner that a distal end portion of the drain cable is exposed from the distal end portion of the shield member.
